Europäisches Patentamt

European Patent Office

Office européen des brevets

(19)

(11) Veröffentlichungsnummer : **0 165 430**
**B1**

(12) **EUROPÄISCHE PATENTSCHRIFT**

(45) Veröffentlichungstag der Patentschrift :
05.04.89

(21) Anmeldenummer : 85105587.1

(22) Anmeldetag : 07.05.85

(51) Int. Cl.⁴ : **A 61 K 33/14**, A 61 L 15/03

(54) Pharmazeutische topische Zubereitungen, Verfahren zu deren Herstellung und Mittel zur Förderung der Wundgranulation und Epithelisation.

(30) Priorität : 07.05.84 DE 3416777
25.09.84 DE 3435113

(43) Veröffentlichungstag der Anmeldung :
27.12.85 Patentblatt 85/52

(45) Bekanntmachung des Hinweises auf die Patenterteilung : 05.04.89 Patentblatt 89/14

(84) Benannte Vertragsstaaten :
AT BE CH DE FR GB IT LI LU NL SE

(56) Entgegenhaltungen :
DE—A— 2 849 570
UNLISTED DRUGS, Band 33, Nr. 1, Januar 1981,
Chatham, New Jersey, US;

(73) Patentinhaber : GÖDECKE AKTIENGESELLSCHAFT
Salzufer 16
D-1000 Berlin 10 (DE)

(72) Erfinder : Niedner, Roland, Dr.
Fillibachstrasse 8
D-7800 Freiburg (DE)
Erfinder : Marmé, Dieter, Prof.
Wildtalstrasse 19
D-7800 Freiburg (DE)
Erfinder : Schöpf, Erwin, Prof.
Immenberg 1
D-7800 Freiburg (DE)

EP 0 165 430 B1

## Beschreibung

Von in vitro Untersuchungen an Zellkulturen ist bekannt, daß Ca$^{2-}$-Ionen einen Einfluß auf die Zellteilung ausüben. (Proc. Nat. Acad. Sci. 70, S. 675-679). Weiterhin ist es bekannt, daß die Depolarisation des Membranpotentials durch Erhöhung der extrazellulären K$^-$-Konzentration erreicht werden kann. Über die Einflüsse dieser Effekte auf die Wundgranulation und Epithelisation ist jedoch bisher nichts bekannt. Umfangreiche Untersuchungen haben aber ergeben, daß weder die bei Zellkulturen als für das Wachstum optimal gefundenen Ca$^{2-}$-Konzentrationen noch höhere Ca$^{2+}$-Konzentrationen einen fördernden oder hemmenden Einfluß auf die Wundheilung haben.

Auch die Zugabe von physiologischen Kaliummengen im extrazellulären Bereich führt zu keiner Förderung der Wundheilung, selbst wenn man gleichzeitig für eine optimale extrazelluläre Ca$^{2-}$-Konzentration sorgt.

Es wurde nun zunächst überraschend gefunden, daß Ca$^{2-}$ und K$^+$ Ionen gemeinsam in ganz bestimmten Konzentrationsverhältnissen eine unerwartet gute und bei idealen Molverhältnissen sogar eine sehr starke Wirkung auf die Wundgranulation und Epithelisation ausüben und daher in Kombination ein wertvolles Mittel zur Förderung der Wundheilung darstellen.

Die erfindungsgemäßen Konzentrationsverhältnisse lassen sich erzielen, wenn man dafür sorgt, daß die Wundoberfläche unmittelbar mit einem wasserhaltigen Elektrolyten in Verbindung gebracht wird, der bezogen auf das in diesem Elektrolyten enthaltene Wasser 20 bis 100 mM Ca$^{2-}$-Ionen und 25 bis 60 mM K$^+$-Ionen enthält.

Der Elektrolyt selbst kann aus Wundsekret und/oder einer zusätzlich auf die Wunde applizierten wässrigen Zubereitung bestehen.

Im ersteren Fall werden physiologisch verträgliche ionisierbare Ca- und K-Salze zusammen mit einem festen Träger im Molverhältnis 1 : 3 bis 4 : 1 in einer solchen Menge auf die Wunde gebracht, daß die Ca- und K-Salze im Wundsekret Lösungen im oben angegebenen Konzentrationsbereich bilden. Da jedoch die Menge des abgegebenen Wundsekrets schwankt und nur ungenau geschätzt werden kann, ist mit Trockengelen, Pudern, Ionenaustauschern, Vliesen, imprägnierten Verbandsmaterialien, Polysacchariden oder ähnlichen trockenen Zubereitungen nicht immer sichergestellt, daß der erfindungsgemäße Konzentrationsbereich erzielt und über eine längere Zeit aufrechterhalten werden kann.

Günstiger sind daher solche Zubereitungen, die zwar in trockener Form hergestellt werden und teilweise auch in dieser Form in den Handel kommen, und die dazu bestimmt sind, vor der Anwendung mit einer relativ genau bestimmten Menge Wasser versetzt zu werden. Diese werden erst nach Aufnahme dieser Wassermenge und erfolgter Dissoziation der Ca$^{2-}$ und K$^-$-Salze auf die Wunde gebracht. Hierzu gehören z. B.

Trockengele gemäß DE-A-28 49 570.

Auch Mischformen, bei denen trockene Träger von vornherein mit Wasser angefeuchtet sind, das Ca$^{2-}$- und K$^+$ Ionen im gewünschten Verhältnis gelöst enthält, sind denkbar. Zu diesen Formen gehören beispielsweise Ionenaustauscher, zur Gelchromatographie geeignete Gele (Molekularsiebe) oder einfache feste, z. B. textile Wundauflagen, die angefeuchtet und anschließend steril eingesiegelt werden.

Die zuletzt genannten Zubereitungen sind an sich bereits typische wässrige Zubereitungen. Hierunter versteht man Zubereitungen bei denen vor allem Wasser als Träger fungiert und Ca$^{2-}$-Ionen sowie K$^+$-Ionen gegebenenfalls neben anderen Hilfs- und oder Wirkstoffen in einer Konzentration von 20 bis 100 mM bzw. 25 bis 60 mM enthält. Der bevorzugte Bereich liegt hier bei 25 bis 35 mM Ca$^{2-}$-Ionen und 35 bis 45 mM K$^-$-Ionen. Ganz besonders bevorzugt ist das Verhältnis von 30 mM Ca$^{2-}$-Ionen und 40 mM K$^-$ Ionen.

Als wässrige Zubereitungen kommen alle pharmazeutischen Zubereitungen in Frage, die einen Wassergehalt aufweisen, der die Einstellung der erfindungsgemäßen molaren Ionenkonzentrationen erlaubt. Neben einfachen wässrigen Lösungen, Lotionen oder Öl in Wasser Emulsionen gehören hierzu auch visköse Lösungen, dispergierte Systeme oder Schäume.

Die oben erwähnten gelartigen Zubereitungen, wie z. B. Polyacrylamid/Agar-Gele sind besonders wundverträglich und daher bevorzugt.

Für die obengenannten Zubereitungen ist ein Verfahren zu Herstellung bevorzugt, das dadurch gekennzeichnet ist, daß man einem wässrigen pharmazeutischen Träger als Wirkstoff in an sich bekannter Weise, gegebenenfalls neben anderen Hilfs- und/oder Wirkstoffen, bezogen auf das enthaltene Wasser, bis zu einer Konzentration von 20 bis 100 (mMol) Ca$^{2+}$ Ionen und 25 bis 60 (mMol) K$^+$-Ionen, Ca$^{2+}$ und K$^+$-Ionen bildende pharmakologisch verträgliche Salze zufügt und diese Ionen in der wässrigen Phase gleichmäßig verteilt.

Als pharmakologisch verträgliche Salze kommen vor allem Chloride und Phosphate infrage, es können jedoch auch andere anorganische oder organische Salze, wie z. B. Citrate, Maleate, Succinate oder ähnliche Salze verwendet werden, sofern sie gewebeverträglich und dissoziierbar sind.

Im Falle wässriger Zubereitungen ist es günstig, die wässrige Phase isoton einzustellen. Dies kann, zwecks Vermeidung von Fremdionen vorzugsweise mittels Glucose erfolgen. Die Effekte von kationischen Elektrolyten, die neben Ca$^{2-}$ und K$^-$ zugegeben werden, sind noch nicht erforscht. In vielen Fällen ist es auch günstig, der wässrigen Phase Tenside zuzufügen, die die Hautpermeabilität für Elektrolyte steigern.

Da die Wirksamkeit der obengenannten Zubereitungen allein auf dem beanspruchten Ionenver-

hältnis von Ca$^{2+}$ und K$^+$-Ionen im extrazellulären Raum beruht, sind selbstverständlich auch solche Zubereitungen voll wirksam, welche die genannten Ionen, als alleinigen Wirkstoff enthalten. In manchen Fällen jedoch ist die Zugabe weiterer Wirkstoffe erwünscht wie z. B. antibiotisch oder fungistatisch wirkende Mittel oder Oberflächenanästhetika.

Bei den Untersuchungen zum Mechanismus der beschriebenen Zubereitungen wurde weiterhin gefunden, daß der positive Effekt auf die Förderung der Wundgranulation und Epithelisation nicht nur bei der Anwesenheit von Ca$^{++}$ und K$^-$-Ionen sondern immer dann auftritt, wenn man die Calciumpermeabilität der Plasmamembran erhöht und somit Ca$^{2+}$-Ionen in die Zelle einströmen können. Dies kann aber nicht nur, wie oben beschrieben, dadurch erreicht werden, daß man in einem pharmakologisch verträglichen Träger gegebenenfalls neben anderen Wirk- und/oder Hilfsstoffen 0,2 bis 10 Gewichtsprozent eines Wirkstoffgemisches, bestehend aus pharmakologisch verträglichen ionisierbaren Ca$^{2+}$ und K$^-$ Salzen im Ca$^{2+}$ : K$^+$-Molverhältnis von 1 : 3 bis 4 : 1 gleichmäßig verteilt, und damit K$^+$-Ionen zum Öffnen der sogenannten Calcium-Kanäle in der Plasmamembran benutzt, sondern auch dadurch daß man anstelle des beschriebenen Wirkstoffgemisches entweder ein Ca$^{2+}$-Ionophor in einer Konzentration von $3 \times 10^{-8}$ bis $3 \times 10^{-6}$ M in Kombination mit Calciumsalzen in einer Konzentration von 0,1-50 mM, oder einen Calcium-Agonisten in einer Konzentration von $10^{-5}$ bis $10^{-9}$ M in Gegenwart von K$^+$-Ionen in einer Konzentration von $5 \times 10^{-3}$ bis $2 \times 10^{-2}$ M und Ca$^{++}$ in einer Konzentration von $10^{-3}$ M bis $3 \times 10^{-2}$ M in einem pharmakologisch verträglichen Träger gleichmäßig verteilt und die erhaltene Zubereitung als Wundheilungsmittel anwendet.

Ca$^{2+}$-Ionophore sind Verbindungen, die die Plasmamembran selektiv für Calcium permeabilisieren.

Ein solches Ionophor ist zum Beispiel das zunächst unter der Bezeichnung A 23 187 bekannt gewordene Antibioticum 6S [6 α(2S*, 3S*), 8 β(R*), 9 β, 11 α]-5-Methylamino-2-[3,9,11-trimethyl-8-[1-methyl-2-oxo-2-(1H-pyrrol-2-yl)-ethyl]-1,7-dioxaspiro [5,5] undec-2-yl] methyl-4-benzoxazolcarbonsäure. [Hœchst Doc. No 8154-1082].

In Kombination mit Calcium wirkt A 23 187 erfindungsgemäß in einer Konzentration von $3 \times 10^{-6}$ bis $3 \times 10^{-8}$ M wobei die Calciumsalze in extrazellulären Raum nur in einer Konzentration von 0,1 bis 50 mM vorhanden sein müssen, um einen ausreichenden Ca$^{2+}$-Zufluß in die Zelle sicherzustellen.

Da es sich mithin prinzipiell um die Calciumversorgung der Zelle durch die Plasmamembran handelt, kann der erfindungsgemäße Effekt der rascheren Wundheilung ebenso durch alle anderen Ca-Ionophore erreicht werden.

Desgleichen wirken Ca-Agonisten, da auch sie die Calciumpermeabilität der Plasmamembran erhöhen. Durch sie werden bei Anwesenheit von Kalium die Calcium-Kanäle geöffnet. Bei der erfindungsgemäßen Anwendung von Ca-Agonisten braucht aber nur sehr wenig Kalium appliziert werden, so daß, besonders bei großflächigen Wunden der Kaliumhaushalt nicht gestört wird. Dies ist eine Verbesserung gegenüber der Anwendung der reinen Ca$^{++}$ und K$^-$ Ionen, wie sie oben beschrieben ist.

Der wirksamste Konzentrationsbereich der Calcium-Agonisten beträgt $10^{-5}$ bis $10^{-9}$ M. Die benötigte Kalium-Konzentration liegt im Bereich von $5 \times 10^{-3}$ bis $2 \times 10^{-2}$ M. Die Calcium-Konzentration sollte zwischen $10^{-3}$ M und $3 \times 10^{-2}$ M liegen.

Ein solcher Calcium-Agonist ist zum Beispiel die Substanz Bay K 8644, von der es bereits bekannt ist, daß sie den Calcium-Zustrom in die Zelle stimuliert [Vgl. z. B. Arzneimittelforschung/ Drug. Res. 33 (II) Nr. 9 (1983) sowie Biochem. and Biophys. Research Communic. (1984) 118, No. 3, S. 842-847].

Es ist als äußerst überraschend zu bezeichnen, daß innerhalb der Zelle Calcium ein wesentlicher Faktor der Wundgranulation ist. Die Calciumkonzentration im Extrazellularraum hat nämlich ohne die erfindungsgemäßen Mittel zur Permeabilitätssteigerung keinerlei Auswirkungen auf die Wundheilung. Weder Entzug von Calcium durch Zugabe eines Calcium-Chelators wie Ethylenglycol-bis (β-amino-ethyl)-N,N,N',N'-tetraessigsäure (EGTA) noch eine Erhöhung der Calciumkonzentration um das zwanzigfache der physiologischen Konzentration im Extrazellularraum führt zu einer Änderung der Wundgranulation. Daraus konnte der Fachmann nur schließen, daß Calciumionen an der Wundgranulation nicht nennenswert beteiligt sind.

Gegenstand der vorliegenden Erfindung ist daher eine pharmazeutische topische Zubereitung zur Förderung der Wundgranulation und Epithelisation, enthaltend einen pharmakologisch verträglichen Träger und einen Wirkstoff, die dadurch gekennzeichnet ist, daß der Träger überwiegend aus Wasser besteht oder in Form eines wasserhaltigen Gels oder als Trockengel vorliegt und daß der Wirkstoff die Calciumpermeabilität der Plasmamembran erhöht und entweder

a) aus einem ionisierbaren Gemisch pharmakologisch verträglicher Ca$^{2+}$ und K$^+$ Salze besteht, wobei die Ca$^{2+}$-Ionen im Träger bezogen auf das enthaltene Wasser in einer Konzentration von 20 bis 100 mM und die K$^-$-Ionen in einer Konzentration von 25 bis 60 mM vorliegen ; oder

b) aus einem Ca$^{2+}$-Ionophor in einer Konzentration von $3 \times 10^{-8}$ bis $3 \times 10^{-8}$ M in Kombination mit Calciumsalzen in einer Konzentration von 0,1 bis 50 mM ; oder

c) aus einem Calcium-Agonisten in einer Konzentration von $10^{-5}$ bis $10^{-9}$ in Gegenwart von K$^-$-Ionen in einer Konzentration von $5 \times 10^{-3}$ bis $2 \times 10^{-2}$ M und Ca$^{2+}$ in einer Konzentration von $10^{-3}$ M bis $3 \times 10^{-2}$ M besteht.

Ein weiterer Gegenstand der Erfindung ist ein Verfahren zur Herstellung einer topischen pharmazeutischen Zubereitung zur Förderung der Wundgranulation und Epithelisation, das dadurch gekennzeichnet ist, daß man in einem pharmakologisch verträglichen Träger gegebenenfalls neben anderen Wirk- und/oder Hilfsstoffen einen Wirkstoff a), b) oder c) gleichmäßig verteilt, sowie die Mittel zur Förderung der Wundgranulation und Epithelisation.

Die Figuren sollen den Gegenstand der Erfindung näher erläutern :

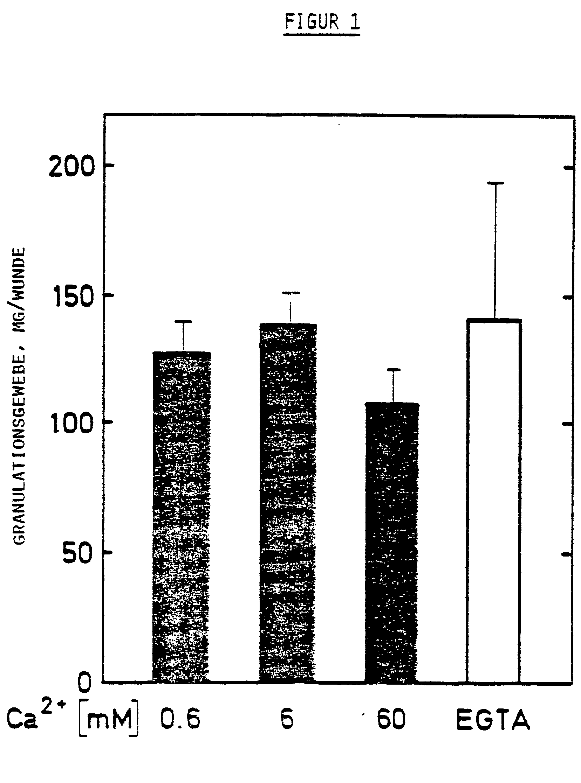

Aus Figur 1 ist ersichtlich, daß die Bildung von Granulationsgewebe im wesentlichen unabhängig ist von der extrazellulären Calcium-Konzentration.

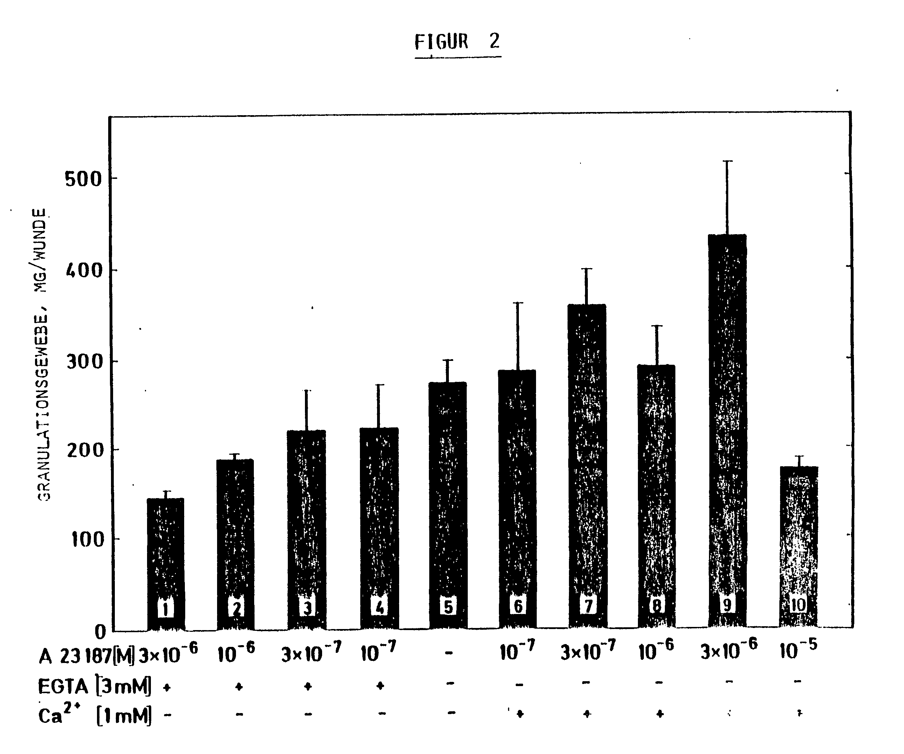

Aus Figur 2 geht hervor, daß der permeabilitätssteigernde Wirkstoff A 23 187 bei Anwesenheit von $Ca^{2+}$-Ionen im Extrazellularraum die Wundgranulation bei einer Konzentration zwischen $10^{-7}$ und $3 \times 10^{-6}$ M, insbesondere bei der Konzentration $3 \times 10^{-6}$ M gegenüber der Vergleichsprobe 5 deutlich steigert. Vermindert man die $Ca^{2+}$-Konzentration in der Zelle durch $Ca^{2+}$-Entzug mittels EGTA im Extrazellularraum und Anwendung von A 23 187 als permeabilitätssteigerndes Mittel, so nimmt die Granulation deutlich ab. Dieser Versuch zeigt, daß die Bildung von Granulationsgewebe, eine Voraussetzung der Wundheilung, vom $Ca^{2+}$-Gehalt der Zellen stark abhängig ist und, daß die Granulation erheblich gesteigert werden kann, wenn man mit den erfindungsgemäßen permeabilitätssteigernden Mitteln den $Ca^{2+}$-Gehalt der Zelle erhöht.

Die den Figuren 1 und 2 zugrundeliegenden Versuche wurden wie folgt durchgeführt :

Es wurde die Rückenhaut von Meerschweinchen bis auf die Faszie durchtrennt. In die Wunde wurde ein Teflonring mit einem Durchmesser von 21 mm eingenäht. Hierdurch sollte der epitheliale Wundverschluß verhindert werden. Auf die Faszie der Rückenmuskulatur, die zum Zeitpunkt der Operation frei von Granulationsgewebe war, wurde während 3 Tagen ein wässriges Polyacrylamid-Agargel, enthaltend die Wirkstoffe in der angegebenen Konzentration aufgetragen. Nach 3 Tagen wenn das Optimum der Granulation erreicht war, wurde mit einem scharfen Löffel das gesamte Granulationsgewebe entnommen, gewogen und histologisch aufgearbeitet. Beim Verfahren a) ausgeführt mit einer Konzentration von 30 mM $Ca^{2+}$-Ionen und 40 mM $K^+$-Ionen, wobei das Gel aus Gründen der Isotonie mit 0,9 Gew.% NaCl versetzt war, kam es zu einem signifikanten Anstieg der Menge des Granulationsgewebes bis zu etwa 180 %. Die histologischen Kontrollen ergaben eine echte Zellvermehrung, nicht etwa nur eine Volumenzunahme der Zellen.

Die folgenden Beispiele dienen der näheren Erläuterung der Erfindung :

Beispiel 1

Isotonische Lösung

In einem 100 ml Meßkolben werden 80 ml gereinigtes Wasser vorgelegt. Unter Rühren mit einem Magnetrührer werden dann 20 mg Benzalkoniumchlorid zugegeben und gelöst. Anschließend gibt man nacheinander 0,3 g Kaliumchlorid, 0,44 g Calciumchlorid (beide Salze gem. Ph. Eur. I.) und 2,62 g Glucose-Monohydrat (Ph. Eur. II). Der Kolben wird im Wasserbad auf 20 °C temperiert und abschließend mit gereinigtem ebenfalls 20 °C warmem Wasser bis zur Eichmarke aufgefüllt. Die Lösung wird dann über ein Membranfilter von 0.2 μm Porenweite sterilfiltriert und steril abgefüllt.

$$[0{,}29822 \text{ g KCl} \sim 40 \text{ mMol K}^-$$
$$0{,}44106 \text{ g CaCl}_2 \sim 30 \text{ mMol Ca}^{2-}]$$

Beispiel 2

Öl-in-Wasser Emulsion

In einem ersten Ansatz werden 7 g einer Mischung bestehend aus gesättigten Fettsäuren, Fettalkoholen, Wollwachs, Mineralölen und nichtionogenen Emulgatoren zusammen mit 2,5 g Polyethylenglykol-Glycerolfettsäureester, 3 g Monoglyceriden der Stearin- und Palmitinsäure, 0,3 g Cetylalkohol und 3,0 g Isopropylpalmitat durch Erwärmen auf 70 °C im Wasserbad homogen geschmolzen.

In einem zweiten Ansatz werden 80 g gereinigtes Wasser unter Rühren mit 3 g Propylenglykol gemischt und auf 70 °C erwärmt. Das so erhaltene Gemisch wird dann mit 0,3 g Kaliumchlorid, 0,44 g Calciumchlorid und 0,2 g eines Konservierungsmittels versetzt. Die erhaltene klare Lösung wird unter Rühren bei 70 °C in den ersten Ansatz einemulgiert. Die so erhaltene Emulsion wird auf 40 °C abgekühlt und der durch Verdunstung erlittene Wasserverlust ergänzt. Die auf 30 °C abgekühlte Emulsion wird dann abgefüllt.

Beispiel 3

Transparentes Flüssigkeitsverbandmaterial (Gelplatte)

Ansatz A :
3,5 g Acrylamid und 0,091 g Bisacrylamid werden in 100 ml gereinigtem Wasser gelöst. Die Lösung wird dann auf 60 °C temperiert.

Ansatz B :
0,3 g Kaliumchlorid und 0,44 g Calciumchlorid werden in 100 ml gereinigtem Wasser gelöst und mit 0,2 g Konservierungsmittel versetzt. Nach Zugabe von 2 g Agar (ÖAB9) wird die Lösung unter Rühren mit einem Magnetrührer zum Kochen gebracht und danach auf 60 °C abgekühlt.

Anschließend werden die Ansätze A und B bei 60 °C unter Rühren gemischt. Danach werden 0,045 g Ammoniumperoxydisulfit und 0,045 g (60 μl) Tetramethylendiamin zugegeben. Nach kurzem intensivem Rühren erfolgt das Ausgießen in Petrischalen, die zuvor im Wärmeschrank auf 60 °C vorgewärmt werden. Die gefüllten Petrischalen werden dann 30 Minuten in einem auf 56 °C eingestellten Wärmeschrank gelagert. Anschließend läßt man auf Raumtemperatur abkühlen und stellt die Schalen mit den erhaltenen transparenten erstarrten Platten 24 Stunden in einen auf 4 °C temperierten Schrank zur Ausreifung. Die so erhaltenen Platten können unmittelbar zur Abdeckung von Wunden eingesetzt werden.

Beispiel 4

Streichfähiges Gel

94 g gereinigtes Wasser werden auf 70 °C erwärmt und mit 0,3 g Kaliumchlorid und 0,44 g Calciumchlorid versetzt. Nach Zugabe von 0,2 g Konservierungsmittel werden in die erhaltene Lösung 5 g Methylhydroxyethylcellulose dispergiert. Dann wird unter Rühren abgekühlt. Nach dem Erkalten erhält man ein hochviskoses mit Luftblasen durchsetztes Gel mit einer Viskosität von 90 Pa.s, das unmittelbar zur Wundbehandlung eingesetzt werden kann.

Beispiel 5

Textile Wundauflage

$4 \times 4$ cm große und 5 mm dicke sterile Mull-Läppchen werden in die sterile isotonische Lösung gemäß Beispiel 1 getaucht und anschließend nur so weit ausgepreßt, daß die Läppchen nicht mehr tropfen. Die so erhaltenen Läppchen werden dann unter sterilen Bedingungen in Polyäthylenfolie eingeschweißt.

Beispiel 6

Streichfähiges Gel mit A 23 187

1 000 g gereinigtes Wasser werden auf 70 °C erwärmt und mit 1,6 mg A 23 187 und 560 mg Calciumchlorid versetzt. Nach Zugabe von 2 g Konservierungsmittel werden in die erhaltene Lösung 50 g Methylhydroxyethylcellulose dispergiert. Dann wird unter Rühren abgekühlt. Nach dem Erkalten erhält man ein hochviskoses mit Luftblasen durchsetztes Gel mit einer Viskosität von 90 Pa.s, das unmittelbar zur Wundbehandlung eingesetzt werden kann.

Beispiel 7

Streichfähiges Gel mit Bay K 8644

1 000 g gereinigtes Wasser werden auf 70 °C erwärmt und mit 1,49 g KCl, 3,3 g $CaCl_2$ und 3,56 mg Bay K 8644 versetzt. Nach Zugabe von 2 g Konservierungsmittel werden in die erhaltene Lösung 50 g Methylhydroxyethylcellulose dispergiert und wie in Beispiel 1 aufgearbeitet.

**Patentansprüche** (für die Vertragsstaaten : BE, CH, DE, FR, GB, IT, LI, LU, NL, SE)

1. Pharmazeutische topische Zubereitung zur Förderung der Wundgranulation und Epithelisation, enthaltend einen pharmakologisch verträglichen Träger und einen Wirkstoff, dadurch gekennzeichnet, daß der Träger überwiegend aus Wasser besteht oder in Form eines wasserhaltigen Gels oder als Trockengel vorliegt und daß der Wirkstoff die Calciumpermeabilität der Plasmamembran erhöht und entweder

a) aus einem ionisierbaren Gemisch pharmakologisch verträglicher $Ca^{2-}$ und $K^-$ Salze besteht, wobei die $Ca^{2+}$-Ionen im Träger bezogen auf das enthaltene Wasser in einer Konzentration von 20 bis 100 mM und die $K^-$-Ionen in einer Konzentration von 25 bis 60 mM vorliegen ; oder

b) aus einem $Ca^{2+}$-Ionophor in einer Konzentration von $3 \times 10^{-8}$ bis $3 \times 10^{-6}$ M in Kombination mit Calciumsalzen in einer Konzentration von 0,1 bis 50 mM ; oder

c) aus einem Calcium-Agonisten in einer Konzentration von $10^{-5}$ bis $10^{-9}$ in Gegenwart von $K^+$-Ionen in einer Konzentration von $5 \times 10^{-3}$ bis $2 \times 10^{-2}$ M und $Ca^{2-}$ in einer Konzentration von $10^{-3}$ M bis $3 \times 10^{-2}$ M besteht.

2. Pharmazeutische Zubereitung gemäß Anspruch 1, dadurch gekennzeichnet, daß diese 30 mM $Ca^{2+}$-Ionen und 40 mM $K^-$-Ionen enthält.

3. Verfahren zur Herstellung einer topischen pharmazeutischen Zubereitung zur Förderung der Wundgranulation und Epithelisation, dadurch gekennzeichnet, daß man in einem pharmakologisch verträglichen Träger gegebenenfalls neben anderen Wirk- und/oder Hilfsstoffen einen Wirkstoff a), b) oder c) nach einem der Ansprüche 1 oder 2 gleichmäßig verteilt.

4. Verfahren gemäß Anspruch 3, dadurch gekennzeichnet, daß der Träger im wesentlichen aus Wasser besteht und daß man diesem gegebenenfalls neben anderen Hilfs- und/oder Wirkstoffen bezogen auf das enthaltene Wasser bis zu einer Konzentration von 20 bis 100 mM $Ca^{2-}$-Ionen und 25 bis 60 mM $K^-$-Ionen zufügt und diese Ionen in der wässrigen pharmazeutischen Unterlage gleichmäßig verteilt.

5. Pharmazeutische Zubereitung gemäß Anspruch 1, dadurch gekennzeichnet, daß das Gel Methylhydroxyethylcellulose enthält.

**Patentansprüche** (für den Vertragsstaat AT)

1. Verfahren zur Herstellung einer pharmazeutischen topischen Zubereitung zur Förderung der Wundgranulation und Epithelisation, enthaltend einen pharmakologisch verträglichen Träger und einen Wirkstoff, dadurch gekennzeichnet, daß der Träger überwiegend aus Wasser besteht oder in Form eines wasserhaltigen Gels oder als Trockengel vorliegt und daß der Wirkstoff die Calciumpermeabilität der Plasmamembran erhöht und entweder

a) aus einem ionisierbaren Gemisch pharmakologisch verträglicher $Ca^{2-}$ und $K^-$ Salze besteht, wobei die $Ca^{2-}$-Ionen im Träger bezogen

auf das enthaltene Wasser in einer Konzentration von 20 bis 100 mM und die K⁻-Ionen in einer Konzentration von 25 bis 60 mM vorliegen ; oder

b) aus einem $Ca^{2-}$-Ionophor in einer Konzentration von $3 \times 10^{-8}$ bis $3 \times 10^{-6}$ M in Kombination mit Calciumsalzen in einer Konzentration von 0,1 bis 50 mM ; oder

c) aus einem Calcium-Agonisten in einer Konzentration von $10^{-5}$ bis $10^{-9}$ in Gegenwart von K⁺-Ionen in einer Konzentration von $5 \times 10^{-3}$ bis $2 \times 10^{-2}$ M und $Ca^{2-}$ in einer Konzentration von $10^{-3}$ M bis $3 \times 10^{-2}$ M besteht, und daß man in einem pharmakologisch verträglichen Träger gegebenenfalls neben anderen Wirk- und/oder Hilfsstoffen einen Wirkstoff a), b) oder c) gleichmäßig verteilt.

2. Verfahren gemäß Anspruch 1, dadurch gekennzeichnet, daß der Träger im wesentlichen aus Wasser besteht und daß man diesem gegebenenfalls neben anderen Hilfs- und/oder Wirkstoffen bezogen auf das enthaltene Wasser bis zu einer Konzentration von 20 bis 100 mM $Ca^{2+}$-Ionen und 25 bis 60 mM K⁻-Ionen zufügt und diese Ionen in der wässrigen pharmazeutischen Unterlage gleichmäßig verteilt.

3. Verfahren gemäß Anspruch 1 oder 2, dadurch gekennzeichnet, daß man die Konzentration des Wirkstoffs auf etwa 30 mM $Ca^{2+}$-Ionen und 40 mM K⁻-Ionen einstellt.

4. Verfahren nach Anspruch 1 bis 3, dadurch gekennzeichnet, daß man den Wirkstoff in einem wasserhaltigen Gel verteilt.

5. Verfahren nach Anspruch 1 bis 3, dadurch gekennzeichnet, daß man den Wirkstoff in einem aus Trockengel bestehenden Träger verteilt.

6. Verfahren nach Ansprich 1 bis 3, dadurch gekennzeichnet, daß man den Wirkstoff in einem Methylhydroxyethylcellulose enthaltenden Gel verteilt.

**Claims** (for the Contracting States : BE, CH, DE, FR, GB, IT, LI, LU, NL, SE)

1. A topical pharmaceutical preparation for the promotion of granulation and epithelization of wounds, containing a pharmacologically compatible carrier and an active substance, characterised by the fact that the carrier mainly consists of water or occurs in the form of a hydrated gel or as a dry gel and that the active substance increases the calcium-permeability of the plasma membrane and comprises either

a) an ionizable mixture of pharmacologically compatible $Ca^{2-}$ and K⁻ salts wherein, the $Ca^{2-}$ ions in the carrier occur at a concentration of 20 to 100 mM in relation to the water contained therein and the K⁻-ions at a concentration of 25 to 60 mM ; or

b) a $Ca^{2-}$-ionophore at a concentration of $3 \times 10^{-3}$ to $3 \times 10^{-6}$ M in combination with calcium salts at a concentration of 0.1 to 50 mM ; or

c) a calcium-agonist at a concentration of $10^{-5}$ to $10^{-9}$ M in the presence of K⁻-ions at a concentration of $5 \times 10^{-3}$ to $2 \times 10^{-2}$ M and $Ca^{2-}$ at a concentration of $10^{-3}$ M to $3 \times 10^{-2}$ M.

2. Pharmaceutical preparation according to claim 1, characterised by the fact that it contains 30 mM $Ca^{2-}$ ions and 40 mM K⁻ ions.

3. Process for the preparation of a topical pharmaceutical preparation for the promotion of the granulation and epithelization of wounds, characterised by the fact that in a pharmacologically compatible carrier, optionally in addition to other active substances and/or excipients, there is uniformly distributed an active substance a), b) or c) according to one of claims 1 or 2.

4. Process according to claim 3, characterised by the fact that the carrier mainly consists of water, and that, in addition to other excipients and/or active substances, and in relation to the water content, concentrations of from 20 to 100 mM $Ca^{2+}$ ions and 25 to 60 mM K⁻ ions are added to it, and that these ions are uniformly distributed in the aqueous pharmaceutical carrier.

5. Pharmaceutical preparation according to claim 1, characterised by the fact that the gel contains methylhydroxyethyl-cellulose.

**Claims** (for the Contracting State AT)

1. Process for the manufacture of a topical pharmacentical preparation for the promotion of the granulation and epithelization of wounds, containing a pharmacologically compatible carrier and an active substance, characterised by the fact that the carrier consists mainly of water or occurs in the form of a hydrated gel or as a dry gel and that the active substance increases the calcium-permeability of the plasma membrane and comprises either

a) an ionizable mixture of pharmacologically compatible $Ca^{2+}$ and K⁻ salts, wherein the $Ca^{2-}$ ions in the carrier occur at a concentration of 20 to 100 mM in relation to the water contained therein and the K⁺ ions at a concentration of 25 to 60 mM ; or

b) a $Ca^{2-}$-ionophore at a concentration of $3 \times 10^{-8}$ to $3 \times 10^{-6}$ M in combination with calcium salts at a concentration of 0.1 to 50 mM ; or

c) a calcium-agonist at a concentration of $10^{-6}$ to $10^{-9}$ M in the presence of K⁻-ions at a concentration of $5 \times 10^{-3}$ to $2 \times 10^{-2}$ M and $Ca^{2+}$ at a concentration of $10^{-3}$ M to $3 \times 10^{-2}$ M ;

and that in a pharmacologically compatible carrier, optionally in addition to other active substances and/or excipients, there is uniformly distributed an active substance a), b) or c).

2. Process according to claim 1, characterised by the fact that the carrier mainly consists of water, and that, in addition to other excipients and/or active substances, and in relation to the water content, concentrations of from 20 to 100 mM $Ca^{2-}$ ions and 25 to 60 mM K⁻ ions are added to it, and that these ions are uniformly distributed in the aqueous pharmaceutical carrier.

3. Process according to claim 1 or 2, characterised by the fact that the concentration of the active substance is set at about 30 mM $Ca^{2+}$ ions and 40 mM $K^-$ ions.

4. Process according to claim 1 to 3, characterised by the fact that the active substance is distributed in a hydrated gel.

5. Process according to claim 1 to 3, characterised by the fact that the active substance is distributed in a carrier consisting of dry gel.

6. Process according to claim 1 to 3, characterised by the fact that the active substance is distributed in a gel containing methylhydroxyethyl-cellulose.

**Revendications** (pour les Etats contractants : BE, CH, DE, FR, GB, IT, LI, LU, NL, SE)

1. Préparation pharmaceutique topique pour l'accélération de la granulation des plaies et la restauration de l'épithélium contenant un support pharmacologiquement compatible et une substance active, caractérisée en ce que le support est principalement constitué d'eau ou se trouve sous la forme d'un gel aqueux ou sous la forme d'un gel sec, et ce que la substance active augmente la perméabilité de la membrane du plasma pour le calcium et qu'elle est constituée soit :

a) d'un mélange ionisable et pharmacologiquement compatible de sels de $Ca^{2+}$ et de $K^+$, les ions $Ca^{2+}$ se trouvant dans le support à une concentration de 20 à 100 mM et les ions $K^+$ à une concentration de 25 à 60 mM par rapport à l'eau contenue ; soit

b) d'un ionophore de $Ca^{2+}$ à une concentration de $3 \times 10^{-8}$ à $3 \times 10^{-6}$ M en association avec des sels de calcium à une concentration de 0,1 à 50 mM, soit

c) d'un agoniste du calcium à une concentration de $10^{-5}$ à $10^{-9}$ en présence d'ions $K^+$ à une concentration de $5 \times 10^{-3}$ à $10^{-2}$ M et $Ca^{2-}$ à une concentration de $10^{-3}$ M à $3 \times 10^{-2}$ M.

2. Préparation pharmaceutique selon la revendication 1, caractérisée en ce que celle-ci contient 30 mM d'ions $Ca^{2-}$ et 40 mM d'ions $K^-$.

3. Procédé de préparation d'une composition pharmaceutique topique destinée à l'accélération de la granulation des plaies et à la restauration de l'épithélium, caractérisé en ce que, dans un support pharmacologiquement compatible, le cas échéant en plus d'autres substances efficaces et/ou substances auxiliaires, on répartit une substance active (a), (b) ou (c) selon l'une quelconque des revendications 1 ou 2.

4. Procédé selon la revendication 3, caractérisé en ce que le support est essentiellement constitué d'eau et en ce que, à ce support, le cas échéant en plus d'autres substances auxiliaires et/ou substances actives, on ajoute jusqu'à une concentration de 20 à 100 mM d'ions $Ca^{2-}$ et de 25 à 60 mM d'ions $K^-$ par rapport à l'eau contenue, et que l'on répartit régulièrement ces ions dans la composition pharmaceutique aqueuse.

5. Préparation pharmaceutique selon la revendication 1, caractérisée en ce que le gel contient de la méthylhydroxy-éthylcellulose.

**Revendications** (pour l'Etat contractant AT)

1. Procédé de préparation d'une composition pharmaceutique topique pour l'accélération de la granulation des plaies et la restauration de l'épithélium contenant un support pharmacologiquement compatible et une substance active, caractérisé en ce que le support est principalement constitué d'eau ou se trouve sous la forme d'un gel aqueux ou sous la forme d'un gel sec, et ce que la substance active augmente la perméabilité de la membrane du plasma pour le calcium et qu'elle est constituée soit :

a) d'un mélange ionisable et pharmacologiquement compatible de sels de $Ca^{2-}$ et de $K^-$, les ions $Ca^{2+}$ se trouvant dans le support à une concentration de 20 à 100 mM et les ions $K^-$ à une concentration de 25 à 60 mM par rapport à l'eau contenue ; soit

b) d'un ionophore de $Ca^{2-}$ à une concentration de $3 \times 10^{-8}$ à $3 \times 10^{-6}$ M en association avec des sels de calcium à une concentration de 0,1 à 50 mM, soit

c) d'un agoniste du calcium à une concentration de $10^{-5}$ à $10^{-9}$ en présence d'ions $K^-$ à une concentration de $5 \times 10^{-3}$ à $2 \times 10^{-2}$ M et $Ca^{2+}$ à une concentration de $10^{-3}$ M à $3 \times 10^{-2}$ M,

et en ce que, dans un support pharmacologiquement compatible, on répartit régulièrement, le cas échéant en plus d'autres substances efficaces et/ou substances auxiliaires, une substance active (a), (b) ou c).

2. Procédé selon la revendication 1, caractérisé en ce que le support est essentiellement constitué d'eau et en ce que l'on y ajoute, le cas échéant en plus d'autres substances auxiliaires et/ou substances actives, jusqu'à une concentration de 20 à 100 mM d'ions $Ca^{2+}$ et 25 à 60 mM d'ions $K^-$ par rapport à l'eau contenue et que l'on répartit régulièrement ces ions dans la préparation pharmaceutique aqueuse.

3. Procédé selon la revendication 1 ou 2, caractérisé en ce que l'on établit la concentration de la substance active environ à 30 mM d'ions $Ca^{2-}$ et 40 mM d'ions $K^+$.

4. Procédé selon les revendications 1 à 3, caractérisé en ce que l'on répartit la substance active dans un gel aqueux.

5. Procédé selon l'une quelconque des revendications 1 à 3, caractérisé en ce que l'on répartit la substance active dans un support constitué de gel sec.

6. Procédé selon l'une quelconque des revendications 1 à 3, caractérisé en ce que l'on répartit la substance active dans un gel contenant de la méthylhydroxyéthylcellulose.

FIGUR 1

FIGUR 2

EP 0 165 430 B1